# EUROPÄISCHE PATENTSCHRIFT

(11) **EP 1 559 365 B1**
(45) Veröffentlichungstag und Bekanntmachung des Hinweises auf die Patenterteilung: **19.11.2008**
(21) Anmeldenummer: 05001860.5
(22) Anmeldetag: 28.01.2005
(51) Int. Cl.: A61B 5/024

(54) **Elektrodenbauteil zur Herzfrequenzmessung, Verfahren zur Befestigung eines Elektrodenbauteils am Körper einer Person**
Electrode structure for heart rate measurement and method for attaching an electrode structure to the human body
Dispositif d'électrode pour la mesure de la fréquence cardiaque et méthode pour sa fixation au corps humain

(30) Priorität: 29.01.2004 DE 102004004699
(43) Veröffentlichungstag der Anmeldung: 03.08.2005
(73) Patentinhaber: Sigma Elektro GmbH, 67433 Neustadt/ Weinstrasse (DE)
(72) Erfinder: Lippert, Stefan, 73733 Esslingen (DE); Marcu, Alexander, 71229 Leonberg (DE)
(74) Vertreter: Wüstefeld, Regine Marie

(56) Entgegenhaltungen:
- US-A- 3 052 233
- US-A- 4 026 278
- US-A- 5 313 952
- US-A- 5 392 783
- US-A- 6 132 337

## Beschreibung

Die vorliegende Erfindung betrifft ein Elektrodenbauteil zur Herzfrequenzmessung bei einer Person mit einem Befestigungsmittel zur lösbaren Festlegung an einem Umfangsband, insbesondere an einem Brustgurt, wobei das Befestigungsmittel integraler Bestandteil des Elektrodenbauteils ist und wobei das an der Person angeordnete Umfangsband im Spannungszustand an der vom Körper der Person wegweisenden Vorderseite des Elektrotlenbauteits anliegt und dieses festklemmt Des weiteren wird ein Verfahren zur Befestigung dieses Elektrodenbauteils am Körper einer Person mittels eines gespannten Umfangsbandes angegeben, wobei die mit mindestens einer Elektrode mit Kontaktfläche ausgestattete Rückseite des Elektrodenbauteils am Körper einer Person anliegt.

Elektradenbauteile zur Herzfrequenzmessung sind aus der Praxis in verschiedenen Ausführengen bekannt Einerseits gibt es f=lektrodenbauteile aus Metall, die mittels Klebeband am Körper befestigt werden, andererseits gibt es Elektrodenbauteile, die neben mindestens einer Elektrode mit Kontaktfläche eine Sender-Einheit aufweisen. Letztere Elektrodenbauteile sind in der Regel mit einem Umfangsband verbindbar und werden am Brustkorb, am Handgelenk oder an einem anderen Körperteil, an dem die Herzfrequenzsignale empfangen werden können, angeordnet Die Sender-Einheit leitet die über die Elektroden erfassten Herzfrequenzsignale an ein Auswerte- und oder Anzeigegerät weiter. Das Auswerte- und / oder Anzeigegerät kann intern, im Elektrodenbauteil selbst, oder extern vorgesehen sein, wobei es dann bspw. am Handgelenk getragen werden kann und die Signalübertragung drahtlos erfolgt.

Ein wichtiges Thema ist die Befestigung des Elektrodenbauteils am Körper einer Person. Ein Umfangsband wird eingesetzt, das mit dem Elektrodenbauteil verbunden wird und dann um den Körper gespannt wird. Ein am Brustumfang zum Einsatz kommendes Elektrodenbauteil ist in Form eines Elektrodengürtels ausgebildet, der in einer Einheitsgröße quer über den Brustkorb geführt ist, an diesem anliegt und an seinen freien Enden mit dem Umfangsband verbunden ist Das Umfangswand besteht in der Regel aus einem elastischen Material und wird - bspw. über eine herkömmliche Schnalle - so über den Brustkorb gespannt, dass die Kontaktflächen der Elektroden des Elektrodengürtels fest anliegen und sich möglichst nicht lockern.

Problematisch ist einerseits die Atemtätigkeit der Person, wobei sich der Brustkorb hebt und senkt und so eine Lockerung der Elektrodenkontaktstelle erfolgen kann. Weiter ist problematisch, dass der Elektrodengürtel für einen schmalen Brustkorb oder einen Kinderbrustkorb zu lang ist und sehr stark gebogen werden muss, damit der für den festen Sitz der Elektrodenkontaktstelle notwendige Spannungszustand erreicht wird. An dem nur in Grenzen flexiblen Elektrodengürtel wirken Rückstellkräfte, die so eine Lockerung der Elektrodenkontaktstellensitzes bewirken.

Mit der voranstehenden Problematik hat sich zum Teil bereits die DE 299 10 633 U1 befasst, wobei es um die Verbesserung einer Verbindungsstelle zwischen Elektrodenbauteil und Umfangsband geht. Dort sollte eine bereits existierende Verbindung per Verbindungskopf, der in eine Aussparung am freien Ende des Elektrodengürtels eingreift, verbessert werden. Die Konzentration lag darauf, das Durchtreten des Verbindungskopfes durch die Aussparung infolge nach außen wirkender Kräfte des Körpers oder durch schmale Körperformen zu verhindern. Grundsätzlich wurde die Verbindungstechnik beibehalten und lediglich eine Stützkonstruktion vorgesehen, die das Durchtreten des Verbindungskopfes durch die Aussparung verhindert.

Die voranstehend beschriebene Verbindungstechnik für einen Elektrodengürtel ist konstruktiv aufwendig und störanfällig, da der Verbindungskopf permanent am Umfangsband angeordnet ist und bei der Pflege und Reinigung desselben eine Abnutzung, Beschädigung oder Verlust erfahren kann. Ebenso kann die Stützkonstruktion an der Aussparung abgenutzt werden. Außerdem bleibt grundsätzlich das Problem erhalten, dass an einem für einen schmalen Brustkorb oder einen Kinderbrustkorb stark gebogenen Elektrodengürtel Rückstellkräfte wirken. Jedenfalls könnte es zu einer Verringerung der Spannung und des Festsitzes des Umfangsbandes und zu einem Spiel zwischen Elektrodenkontaktstelle und Körper kommen, was zu einer Lockerung des Elektrodenkontaktstellensitzes oder gar zu einem Verrutschen des Elektrodenbauteils führen kann und sich negativ auf die Messung auswirken kann. Schließlich ist die Handhabung aufwendig, denn zwei freie Enden eines Umfangsbandes müssen mit zwei freien Enden eines Elektrodengürtels verbunden werden.

Außerdem kosten die Verbindungsvorgänge einige Zeit. Zur Art und Weise der Befestigung des Elektrodengürtels am Körper wird davon ausgegangen, dass zunächst einseitig die Verbindung zwischen Umfangsband und Elektrodengürtel hergestellt wird, dann das Umfangsband mit dem Elektrodengürtel um den Körper gelegt und dann die zweite Verbindung hergestellt wird. Schließlich wird über eine herkömmliche Schnalle eine Spannung vorgenommen. Diese Vorgehensweise erfordert viele Handgriffe und mehrere Vorfixierungsschritte bis es schließlich zur tatsächlichen Betriebsstellung am Körper kommt Insgesamt sind auch die Herstellungskosten der bekannten Gesamtanordnung hoch, da das Umfangsband mit Verbindungsteilen für die am Elektrodengürtel befindlichen Verbindungsteile konfektioniert werden muss.

Aus der US 6 132 337 die die Merkmale des Oberbegriffs des Anspruchs 1 offenbart, ergibt sich ein Elektrodengürtel, wobei das Band vor der zum Körper weisenden Elektrode entlang geführt ist. Die dortigen Befestigungsmittel sind der Elektrode zuzuordnen und bilden eine Öse, durch die das Band hindurchgeführt ist Die Befestigung erfolgt durch Festklemmen. Die Handhabung des bekannten Elektrodengürtels ist einigermaßen aufwendig, da das Elektrodenbauteil zunächst auf das Umfangsband aufgezogen werden muss. Eine Entfernung des Elektrodenbauteils vom Umfangsband ist jedenfalls immer dann notwendig, wenn dieses der Reinigung unterzogen wird. Anschließend erfolgt wieder die Konfektionierung, wobei die Verbindungsvorgänge einige Zeit kosten. Diese Vorgehensweise erfordert viele Handgriffe bis es schließlich zur tatsächlichen Betriebsstellung am Körper kommt.

Die US 5 313 952 offenbart einen Elektrodengürtel mit einem Netzgürtel und einem Schaumstreifen, der zwischen zwei Außenstreifen angeordnet ist. Im Elektrodengürtel sind viereckige Ausnehmungen vorgegeben. Ein Befestigungsmechanismus verbindet die Enden des Elektrodengartels, Das Elektrodenbauteil umfasst eine zum Körper weisende Scheibe, deren Außenseite Empfängerelemente zur Weiterleitung der Herzfrequenz zugeordnet sind. Das Empfängerelement hat einen quadratischen Querschnitt wie die Ausnehmung des Gürtels. Durch die Verbindung zur innenliegenden Elektrodenscheibe mit den den Gürtel im Bereich der Ausnehmungen durchgreifenden Empfängerbautell wird das Elektrodenbauteil ausgebildet und die Fixierung erreicht Das bekannte Elektrodenbauteil ist konstruktiv aufwendig und erfordert eine Vielzahl von Handgriffen bei der Befestigung am Brustband sowie eine spezielle Ausgestaltung des letzteren.

Ausgehend von dem vorbekannten Stand der Technik liegt der Erfindung die Aufgabe zugrunde, ein Elektrodenbauteil und ein Verfahren zur Befestigung eines Elektrodenbauteils der in Rede stehenden Art anzugeben, wobei eine einfache und schnelle Handhabung im Hinblick auf die Befestigung am Körper ermöglicht wird.

Die voranstehende Aufgabe wird im Hinblick auf das Elektrodenbauteil durch die Merkmale des Patentanspruches 1 gelöst. Danach ist eine Elektrodenbauteil der in Rede stehenden Art derart ausgestaltet und weitergebildet, dass das Elektrodenbauteil zumindest bereichsweise im Querschnitt als L-Profil und / oder als C-Profil ausgebildet ist.

Zunächst ist erkannt worden, dass ein Elektrodenbauteil eine einfache und schnelle Handhabung im Hinblick auf die Befestigung am Körper ermöglicht, wenn es selbst im Sinne eines Befestigungsmittels ausgebildet ist und jedenfalls im Spannungszustand des Umfangsbandes eine Verbindung zwischen Umfangsband und Elektrodenbauteil besteht Dies schließt natürlich nicht aus, dass an der vom Körper der Person wegweisenden Vorderseite des Elektrodenbauteils und am Umfangsband bspw. Mittel zur Ausbildung einer Nadel-Filz-Verbindung vorgesehen sein könnten. Hauptgedanke ist aber, dass das gespannte Umfangsband das Elektrodenbauteil festklemmt und zwar so, dass die Rückseite des Elektrodenbauteils mit der Elektrodenkontaktstelle, den Körper kontaktiert Außer der einfachen Handhabung, dass kurzerhand das Elektrodenbauteil unter das gespannte Umfangsband geklemmt wird, bringt das erfindungsgemäße Elektrodenbauteil noch weitere Vorteile mit sich. Bei einer entsprechenden Formgebung kann das gleichzeitig als Befestigungsmittel dienende Elektrodenbauteil bewirkten, dass das Umfangsband selbst keinerlei Befestigungsmittel aufweisen muss. Es würde dann ein einfaches Elastikband mit Schnalle ausreichen, das separat gereinigt werden kann und auch ein Verschleiß im Hinblick auf die Verbindungsmittel wäre dann irrelevant. Ein weiterer Vorteil eröffnet sich bei der Hertfrequenzmessung über den Brustbereich mit einem Elektrodenbauteil im Sinne eines Elektrodengürtels, das eine Sender-Einheit enthält, im Hinblick auf die Lorkerungsproblematik. Durch eine geschickte Formgebung kann von der bisher bekannten Elektrodengürtelform abgewichen werden und die Verbiegung desselben infolge Körperkraft oder schmalerer Körperformen spielt keine Rolle mehr

Efindungsgemäß ist das Elektrodenbauteil zumindest bereichsweise im Querschnitt als L-Profil und/oder als c-Profil ausgebildet Durch die Profilierung kann sich das Umfangsband entweder gegen den unteren Schenkel des Profils abstützen, oder - bei einem C-Profil - auch nach oben eine Begrenzung erfahren. Bei einer durch den Körper der Person gedachten Längsachse wäre bei einem C-Profil eine Verschiebesicherung des Umfangsbandes in Richtung parallel zur gedachten Längsachse gegeben.

Damit die durch das gespannte Umfangsband auf den Körper einwirkende Kraft zum Festklemmen des Elektrodenbauteils ausgenutzt werden kann, wird dieses zwischen Umfangsband und Körper angeordnet Es ist zweckmäßig, wenn das Umfangsband von dem Elektrodenbauteil zumindest bereichsweise geführt ist. Auf diese Weise kann verhindert werden, dass das Umfangsband bezüglich des Elektrodenbauteils seine Lage verändert. Voranstehendes bezieht sich bspw. auf den Fall, dass das über die Schnalle in herkömmlicher Weise gespannte Umfangsband durch zufällige mechanische Einwirkung vom Elektrodenbauteil entfernt werden sollte. Außerdem wird bspw. dem Fall Rechnung getragen, dass sich bei Anwendung auf einer Herzfrequenzmessung im Bereich des Brustkorbes sich dieser durch die Atemtätigkeit im Umfang verändert. Dieser Vorgang geht mit einer Bewegung des Umfangbandes einher, wobei es dann darauf ankommt, dass das Umfangsband während dieser Bewegung seine Lage an dem am Körper fest anliegenden Elektrodenbauteil beibehält. Die Führung muss demzufolge so beschaffen sein, dass eine wie auch immer geartete Bewegung des Elastikbandes weitgehend ohne Reibungsverluste verlauft.

Die Profilierung des Elektrodenbauteils könnte dahingehend erweitert werden, dass an den Schenkeln des L- oder C-Profils ein unterer Randbereich vorgesehen ist. Dieser unterer Randbereich könnte sich in etwa parallel zur Vorderseite des Elektronikbauteils erstrecken. Bei einem C-Profil könnte des weiteren ein oberer Randbereich vorgesehen sein der sich ebenfalls, wie der untere Randbereich, in etwa parallel zur Vorderseite des Elektrodenbauteils erstrecken könnte. Auf diese Weise wird eine Bewegung des Umfangsbandes auch in radialer Richtung - ausgehend von der gedachten Längsachse bezüglich des Körpers der Person - gestoppt und es kommt zu keiner ungewollten Lösung des Umfangsbandes aus dem als Befestigungsmittel ausgebildeten Elektrodenbauteil. Im Hinblick auf das C-Profil wäre es denkbar, dieses formgebungsmäßig nahezu in Richtung eines O-Profils mit Einführschlitz für das Umfangsband auszubilden. In diesem Zusammenhang ist offensichtlich, dass das Elektrodenbauteil auch rohrförmig ausgebildet sein könnte, wobei das Umfangsband kurzerhand durch die beiden Durchtrittsöffnungen der Rohrform durchgezogen ist. In diesem Falle der nicht Bestandteil der vorliegender Erfindung ist, würde das Umfangsband im gespannten Zustand an der vom Körper der Person wegweisenden innenliegenden Vorderseite des Elektrodenbauteils anliegen.

Zur Gewährleistung der Funktionstüchtigkeit der durch die Profilierung erfüllten Führungs- und Haltefunktion würde es ausreichen, wenn die Längenabmessung des unteren / oberen Randbereiches nur einen Teil der Gesamtlänge des Elektrodenbauteils ausmacht. Dabei könnten der obere und der untere Randbereich voneinander in Längsrichtung beabstandet sein. Insbesondere die bereichsweise vorhandene C-Profilierung könnte sich über die Längsabmessung des Elektrodenbauteils so gestalten, dass eine bequeme Einbringöffnung für das Umfangsband entsteht.

Wie bereits erwähnt, ist an der zum Körper der Person weisenden Rückseite des Elektrodenbauteils mindestens eine Elektrode mit einer Kontaktfläche vorgesehen. Bei einem für die Herzfrequenzmessung über dem Brustumfang vorgesehenen Elektrodenbauteil könnten insgesamt drei Elektroden vorgesehen sein, wobei zwei für den Bereich des Herzens, vornehmlich für die linke Körperhälfte bis Mitte bestimmt sind. Gemäß einer an den aus dem gattungsbildenden Stand der Technik angelehnten Ausführungsform könnte das Elektrodenbauteil in elektrischer Verbindung zu einer Senderelektronik stehen. Wie der aus dem gattungsbildenden Stand der Technik bekannte Elektrodengürtel, könnte auch das Elektrodenbauteil gemäß der vorliegenden Erfindung mit einem zweiten solchen Elektrodenbauteil und der Senderelektronik gekoppelt sein. Insgesamt könnten die beiden Elektrodenbauteile gemeinsam mit der in einem Gehäuse angeordneten Senderelektronik eine Mess- und Übertragungseinheit ausbilden. Dabei könnten die beiden Elektrodenbauteile und die Senderelektronik fest verbunden sein. Bezüglich einer Variante der Mess- und Übertragungseinheit mit drei Elektroden könnten zwei für den Herzbereich vorgesehene Elektroden einem Elektrodenbauteil zugeordnet sein und die dritte Elektrode dem benachbarten Elektrodenbauteil.

Eine weitere Ausführungsform der Erfindung ist gegenüber dem bekannten Elektrodengürtel, wobei Verbindungsmittel, Elektrodenbauteil, Senderelektronik, Elektrodenbauteil, Verbindungsmittelist sukzessive in einer Reihe angeordnet sind und deshalb eine große Baulänge ergeben, weitergebildet. Und zwar könnten kleinere Baugrößen der voranstehend beschriebenen Mess- und Übertragungseinheit erreicht werden, in dem sich die beiden Elektrodenbauteile teilweise parallel zur Senderelektronik, bzw. zu dessen Gehäuse erstrecken. Die Elektrodenbauteile müssen also nicht - wie beim E-lektrodengürtel - beidseitig von der Senderelektronik angeordnet sein, sondern überlagern diese. Die dadurch erhaltene wesentlich geringere Baugröße der Mess- und Übertragungseinheit ist vor allen Dingen auf Personen mit geringem Brustumfang oder Kinder anwendbar. In diesem Zusammenhang ist die Anpassung der unter Verwendung des erfindungsgemäßen Elektrodenbauteils ausgebildeten Mess- und Übertragungseinheit an beliebige Körpergrößen oder Körperteilumfänge hervorzuheben.

Gemäß der die Mess- und Übertragungseinheit aus zwei erfindungsgemäßen Elektrodenbauteilen und der Senderelektronik betreffenden Ausführungsform ist es im Hinblick auf den Verlauf des Umfangsbandes von Vorteil, wenn sich die beiden Elektrodenbauteile teilweise parallel zur Senderelektronik bzw. dessen Gehäuse erstrecken und einen Abstand zueinander aufweisen, der zur Aufnahme des Umfangsbandes geeignet ist.

Geht man von einem teilweise im Sinne eines C-Profils ausgestalteten Elektrodenbauteil aus, so bezieht sich der Abstand zur Senderelektronik bzw. zum Gehäuse auf das Basisteil des C-Profils. Das gespannte Umfangsband liegt an der Vorderseite des Basisteils an, während die Schenkel mit den Randbereichen das Umfangsband umgreifen. Im Anschluss erstreckt sich das Umfangsband an der Rückseite der Senderelektronik bzw. des Gehäuses, jedoch beabstandet zum Körper der Person. Schließlich erstreckt sich das Umfangsband wieder an der Vorderseite des Basisteils des zweiten Elektrodenbauteils.

Das für die Befestigung des erfindungsgemäßen Elektrodenbauteils unerlässliche Umfangsband könnte in herkömmlicher Weise aus einem textilen, elastischen Material bestehen und auf die jeweilige Körperdimension einstellbar sein. In materialmäßiger Hinsicht könnte das Elektrodenbauteil hinsichtlich seiner Trag-, Befestigungs-, Halte- und Führungsfunktion ausübenden Teile aus einem nichtleitenden Kunststoff bestehen. Die dem Elektrodenbauteil zugeordnete Elektrode ist dagegen aus elektrisch leitendem Material bereitzustellen, wobei ein Kunststoff, wie ABS, oder alternativ auch Metall in Betracht kommt. Eine Metallelektrode könnte in Form einer Metallplatte als Hautkontaktstelle ähnlich wie bei einem EKG-Gerät ausgebildet sein. Das Gehäuse zur Aufnahme der Senderelektronik könnte aus festem Kunststoff bestehen. Insgesamt könnte eine zwei erfindungsgemäße Elektrodenbauteile enthaltende Mess- und Übertragungseinheit als einheitliche Bauform vorliegen, wobei bspw. eine unlösbare Verbindung zwischen dem Gehäuse und den beiden Elektrodenbauteilen bspw. durch Verschweißen hergestellt ist.

Die eingangs formulierte Aufgabe wird des weiteren durch die Merkmale des Verfahrensanspruches 18 gelöst. Das in Rede stehende Verfahren ist dadurch weitergebildet, dass zunächst nur das Umfangsband um den Körper der Person gespannt wird und dass danach das Elektrodenbauteil am Umfangsband eingehängt wird, wobei das Umfangsband das Elektrodenbauteil festklemmt.

Erfindungsgemäß ist erkannt worden, dass mehrere Fixierschritte, mehrere Verbindungsschritte bezüglich des aus dem gattungsbildenden Stand der Technik bekannten Verbindens der freien Enden des Umfangsbandes mit den freien Enden eines Elektrodengürtels auf insgesamt zwei Verfahrensschritte reduziert werden kann, wenn kurzerhand das Umfangsband um den Körper gespannt wird und danach das Elektrodenbauteil zwischen Körper und Umfangsband gelangt und festgeklemmt wird.

Konkret kann das Elektrodenbauteil so beschaffen sein, dass es in das Umfangsband quasi eingehängt wird. Bei der erfindungsgemäsen Ausführungform, wobei das Elektrodenbauteil zumindest teilweise als C-Profil mit einem oberen und unteren, das Umfangsband umgreifenden Randbereich ausgebildet ist, ist das Einhängen besonders gut realisierbar. Durch die Spannung des Umfangsbandes, wird das Elektrodenbauteil jedenfalls am Körper gehalten. Das sogenannte Einhängen des Elektrodenbauteils ist auch dann möglich, wenn dieses Bestandteil einer Mess- und Übertragungseinheit ist. Dabei sind zwei Elektradenbauteile über ein die Senderelektronik enthaltendes Gehäuse miteinander verbunden. Bei dieser Bauform erstrecken sich das die Senderelektronik enthaltende Gehäuse sowie umgreifende Randbereiche der Elektrodenbauteile vor der Vorderseite des gespannten Umfangsbandes. Der Einhängvorgang findet demnach so statt, dass die Mess- und Übertragungseinheit auf den oberen Randbereich des Umfangsbandes aufgesteckt wird und dieses dann über den unteren Randbereich der Elektrodenbauteile hinweg gleitet und an der vom Körper wegweisenden Vorderseite der Elektrodenbauteile sowie an der Rückseite des Gehäuses für die Senderelektronik anliegt.

Eine alternative Befestigungsmöglichkeit, die nicht Bestandteil der rodiegenden Erfindung ist, besteht darin, dass das Elektrodenbauteil auf das Umfangsband aufgezogen wird und mit diesem um den Körper der Person gespannt wird, wobei das Umfangsband das Elektrodenbauteil festklemmt Bei dieser Art von Befestigung sind ebenfalls nur zwei Verfahrensschritte erforderlich, nämlich Aufziehen des Elektrodenbauteils auf das Umfangsband und Umlegen/Festspannen desselben. Auch bei diesem Verfahren versteht sich von selbst, dass dieses auch im Hinblick auf ein aus zwei verbindungsgemäßen Elektrodenbauteilen und einer Senderelektronik aufgebauten Mess- und Übertragungseinheit durchführbar ist

Das erfindungsgemäße Verfahren reduziert die aus dem Stand der Technik bekannten zahlreichen Handgriffe und Vorfixierungsschritte bis zur tatsächlichen Betriebsstellung des Elektrodenbauteils oder einer Mess- und Übertragungseinheit am Körper. Dadurch wird einerseits Zeit gespart und andererseits die Handhabung enorm erleichtert.

Es gibt nun verschiedene Möglichkeiten, die Lehre der vorliegenden Erfindung in vorteilhafter Weise auszugestalten und weiterzubilden. Dazu ist einerseits auf die Patentansprüche, andererseits auf die nachfolgende Erläuterung von zwei Ausführungsbeispielen der Erfindung anhand der Zeichnung zu verweisen. In Verbindung mit der Erläuterung des angeführten Ausführungsbeispiels der Erfindung anhand der Zeichnung werden im allgemeinen bevorzugte Ausgestaltungen und Weiterbildungen der Lehre erläutert. In der Zeichnung zeigen
- Fig. 1: in schematischer Darstellung, eine Vorderansicht eines ersten Ausführungsbeispiels eines erfindungsgemäßen Elektrodenbauteils,
- Fig. 2: in schematischer Darstellung, einen Schnitt entlang der Linie A-A des des Gegenstandes aus Fig. 1, jedoch mit Umfangsband,
- Fig. 3: in schematischer Darstellung die Anordnung des Gegenstandes aus den Fig. 1 und 4 am Körper einer Person,
- Fig. 4: in schematischer Perspektivdarstellung, eine Vorderansicht eines zweiten Ausführungsbeispiels eines erfindungsgemäßen Elektrodenbauteils, umfassend eine Senderelektronik in einem Gehäuse und ein weiteres Elektrodenbauteil im Sinne einer Mess- und Übertragungseinheit und
- Fig. 5: in schematischer Perspektivdarstellung, eine Rückansicht des Gegenstandes aus Fig. 4.

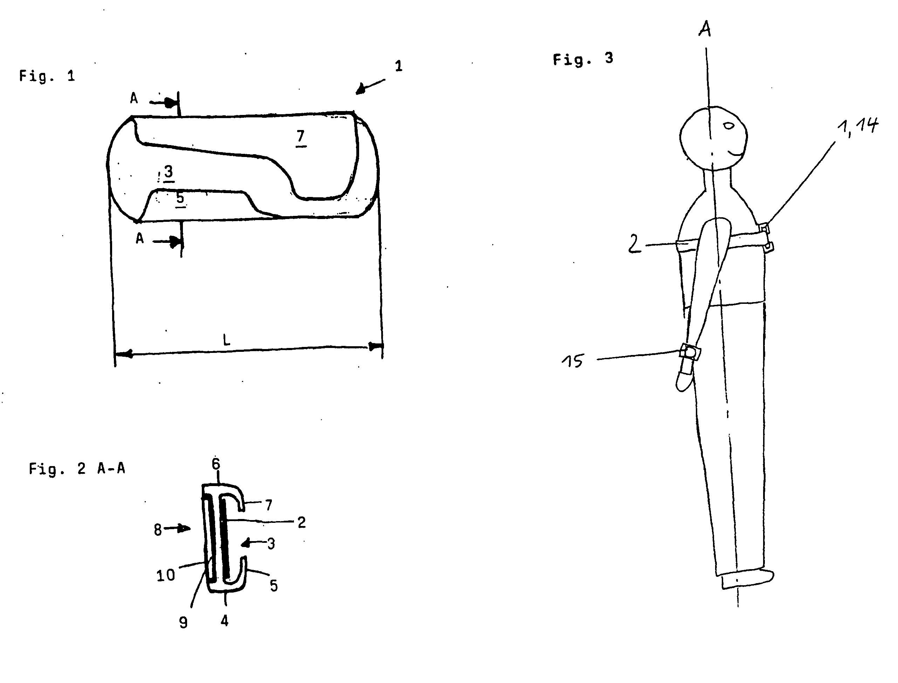

Die Figuren 1 - 5 zeigen zwei Ausführungsbeispiele für ein Elektrodenbauteil 1 zur Herzfrequenzmessung bei einer Person. Das Elektrodenbauteil 1, 11 kann an einem Umfangsband 2 lösbar festgelegt werden. Bei beiden Ausführungsbeispielen liegt das Umfangsband 2 in Form eines Brustgurts vor.

Erfindungsgemäß bildet das Elektrodenbauteil 1, 11 selbst das Befestigungsmittel zur lösbaren Festlegung am Umfangsband 2 aus bzw. das Befestigungsmittel ist integraler Bestandteil des Elektrodenbauteils 1, 11. Wie sich aus Fig. 3 deutlich ergibt, liegt das an der Person angeordnete Umfangsband 2 im Spannungszustand an der vom Körper wegweisenden Vorderseite 3 des Elektrodenbauteils 1 an und klemmt dieses fest.

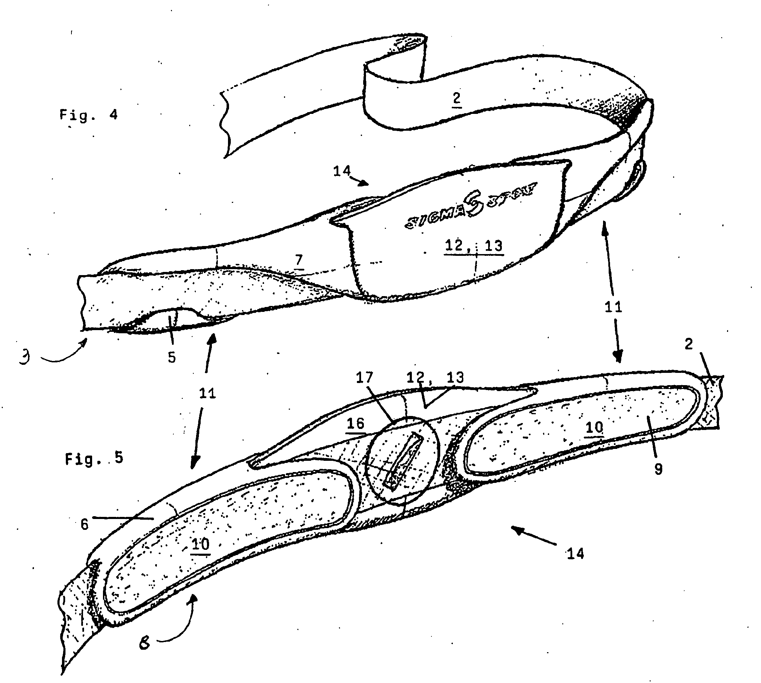

In den Figuren 4 und 5 ist dargestellt, dass das Umfangsband 2 von dem Elektrodenbauteil 1, 11 bereichsweise geführt, gehalten, getragen ist bzw. am Elektrodenbauteil 1, 11 eine lösbare Festlegung erfährt.

In Figur 2 ist dargestellt, dass das Elektrodenbauteil 1 zur Ausbildung oder Integration eines Befestigungsmittels im Querschnitt bereichsweise im Sinne eines C-Profils ausgebildet ist. Der untere Schenkel 4 des C-Profils weist einen Randbereich 5 auf, der sich in etwa parallel zur Vorderseite 3 des Elektrodenbauteils 1 erstreckt. Die Längenabmessung des unteren Randbereiches 5 macht - wie sich aus Figur 1 ergibt - nur einen Teil der Gesamtlänge L des Elektrodenbauteils 1 aus. Der obere Schenkel 6 des C-Profils weist einen oberen Randbereich 7 auf, der sich ebenfalls in etwa parallel zur Vorderseite 3 des Elektrodenbauteils 1 erstreckt. Wie der untere Randbereich 5 erstreckt sich auch der obere Randbereich 7 nur über einen Teil der Gesamtlänge L des Elektrodenbauteils 1. Damit ein bequemes Einhängen des Elektrodenbauteils 1 in den am Körper gespannten Umfangsband 2 möglich ist, sind der obere und der untere Randbereich 5, 7 voneinander beabstandet. Aus Figur 3 wird deutlich, dass durch die Schenkel 4, 6 des C-Profils und durch die Randbereiche 5, 7 nicht nur die Befestigung des Elektrodenbauteil 1, 11 am Umfangsband 2, sondern auch eine Lagesicherung des Umfangsbandes 2 erreicht wird. Die Lagesicherung wird durch die Schenkel 4, 6 in einer Richtung koaxial zu einer gedachten Längsachse A durch den Körper einer Person und durch die Randbereiche 5 und 7 in radialer Richtung zur Längsachse A realisiert.

Die Figuren 2 und 5 zeigen, dass an der zum Körper weisenden Rückseite 8 des Elektrodenbauteils 1, 11 eine Elektrode 9 mit einer Kontaktfläche 10 vorgesehen ist.

Gemäß dem in den Figuren 4 und 5 gezeigten zweiten Ausführungsbeispiel steht das erfindungsgemäße Elektrodenbauteil 1 gemeinsam mit einem weiteren Elektrodenbauteil 11 in elektrischer Verbindung zu einer Senderelektronik 12 die in einem Gehäuse 13 angeordnet ist. Die beiden Elektrodenbauteile 1 und 11 bilden gemeinsam mit der im Gehäuse 13 angeordneten Senderelektronik 12 eine Mess- und Übertragungseinheit 14 aus. Die Mess- und Übertragungseinheit 14 leitet die über die Kontaktflächen 10 der Elektroden 9 empfangenen Herzfrequenzsignale an ein Anzeige- und Auswertegerät 15 weiter, welches hier - wie in Figur 3 gezeigt - am Handgelenk getragen wird. In Figur 3 ist also nicht nur das Elektrodenbauteil 1, sondern auch die Mess- und Übertragungseinheit 14 umfassend das weitere Elektrodenbauteil 11, die Senderelektronik 12, sowie das für die Senderelektronik 12 vorgesehene Gehäuse 13, rein symbolisch dargestellt.

Eine geringe Baugröße der gesamten Sendereinheit 14 wird erreicht, indem sich die beiden Elektrodenbauteile 1, 11 teilweise parallel zur Senderelektronik 12 bzw. zum Gehäuse 13 erstrecken und dieses überlappen. Damit die Sendereinheit 14 in das gespannte Umfangsband 2 eingehängt werden kann, ist es erforderlich, dass die Vorderseiten 3 der Elektrodenbauteile 1 und 11 in radialer Richtung bezogen auf die in Fig. 3 gezeigte Längsachse A beabstandet zur Senderelektronik 12 bzw. zum Gehäuse 13 erstreckt. Im Abstand zwischen der Vorderseite 3 der Elektrodenbauteile 1 und 11 und der Senderelektronik 12 bzw. dem Gehäuse 13 ist das an der Person angeordnete Umfangsband 2 im Spannungszustand entlanggeführt, liegt an der zum Körper der Person hinweisenden Rückseite 16 des Gehäuses 13 an und presst die Kontaktflächen 10 der Elektroden 9 gegen den Körper.

Das Umfangsband 2 besteht aus einem textilen, elastischen Material. Das Elektrodenbauteil 1, 11 besteht hinsichtlich seiner durch das C-Profil mit den Randbereichen 5 und 7 gebildeten Trag-, Befestigungs-, Halte- und Führungsanteile aus einem nicht leitenden Kunststoff. Die dem Elektrodenbauteil 1, 11 zugeordnete Elektrode 9 besteht aus elektrisch leitendem Kunststoff. Das Gehäuse 13 ist aus einem festen, robusten Kunststoff gefertigt und ist unlösbar mit den beiden Elektrodenbauteilen 1, 11 verbunden. Wegen der Übertragung der Herzfrequenzsignale besteht selbstverständlich eine elektrische Verbindung zwischen der jeweiligen Elektrode 9 und der Senderelektronik 12.

Die Handhabung des erfindungsgemäßen Elektrodenbauteils 1 kann auf zwei unterschiedliche Weisen erfolgen. Zum einen wird zunächst das Umfangsband 2 um den Körper der Person gespannt und danach das Elektrodenbauteil 1 bzw. die Mess- und Übertragungseinheit 14 mit den beiden Elektrodenbauteilen 1 und 11 zwischen Körper und Umfangsband 2 verbracht, wobei das Umfangsband 2 das Elektrodenbauteil 1 bzw. die Mess- und Übertragungseinheit 14 festklemmt. Das Elektrodenbauteil 1 oder die Mess- und Übertragungseinheit 14 müssen demnach nur noch am Umfangsband 2 eingehängt werden und zwar so, dass die Randbereiche 5, 7 das Umfangsband 2 übergreifen. Eine weitere Handhabungsmöglichkeit besteht darin, dass das Elektrodenbauteil 1 oder die Mess- und Übertragungseinheit 14 auf das Umfangsband 2 aufgezogen wird und mit diesem um den Körper der Person gespannt wird. Auch hierbei klemmt das Umfangsband 2 das Elektrodenbauteil 1 oder die Mess- und Übertragungseinheit 14 fest.

Mit 17 ist ein Verschluss bezeichnet, der eine Aufnahme für eine Batterie freigibt, wenn er bspw. mittels Geldstück geöffnet wird.

Hinsichtlich weiterer, in den Figuren nicht gezeigter Merkmale wird auf den allgemeinen Teil der Beschreibung verwiesen.

Abschließend sei darauf hingewiesen, dass die erfindungsgemäße Lehre nicht auf die voranstehend erörterten Ausführungsbeispiele eingeschränkt ist. Die Erfindung ist in den Patentansprüchen definiert. Zudem kann bei einer Mess - und Übertragungseinheit, die das erfindungsgemäße Elektrodenbauteil enthält, eine Bauform gefunden werden, die eine Unterteilung in Senderelektronik und Elektrodenbauteile äußerlich nicht erkennen lässt.

## Patentansprüche

1. Elektrodenbauteil zur Herzfrequenzmessung bei einer Person mit einem Befestigungsmittel (4, 5, 6, 7) zur lösbaren Festlegung an einem Umfangsband (2), insbesondere an einem Brustgurt, wobei das Befestigungsmittel (4, 5, 6, 7) integraler Bestandteil des Elektrodenbauteils (1) ist und wobei das an der Person angeordnete Umfangsband (2) im Spannungszustand an der vom Körper der Person wegweisenden Vorderseite (3) des Elektrodenbauteils (1) anliegt und dieses festklemmt,
**dadurch gekennzeichnet,**
**dass** das Elektrodenbauteil (1) zumindest bereichsweise im Querschnitt als L-Profil und / oder als C-Profil ausgebildet ist.

2. Elektrodenbauteil nach Anspruch 1, **dadurch gekennzeichnet, dass** das Umfangsband (2) von dem Elektrodenbauteil (1) zumindest bereichsweise geführt ist.

3. Elektrodenbauteil nach Anspruch 1 oder 2, **dadurch gekennzeichnet, dass** der untere Schenkel (4) des L- oder C-Profils einen unteren Randbereich (5) aufweist, der sich in etwa parallel zur Vorderseite (3) des Elektrodenbauteils (1) erstreckt.

4. Elektrodenbauteil nach Anspruch 3, **dadurch gekennzeichnet, dass** die Längenabmessung des unteren Randbereiches (5) einen Teil der Gesamtlänge (L) des Elektrodenbauteils (1) ausmacht.

5. Elektrodenbauteil nach einem der Ansprüche 1 bis 4, **dadurch gekennzeichnet, dass** der obere Schenkel (6) des C-Profils einen oberen Randbereich (7) aufweist, der sich in etwa parallel zur Vorderseite (3) des Elektrodenbauteils (1) erstreckt.

6. Elektrodenbauteil nach Anspruch 5, **dadurch gekennzeichnet, dass** die Längenabmessung des oberen Randbereiches (7) einen Teil der Gesamtlänge (L) des Elektrodenbauteils (1) ausmacht.

7. Elektrodenbauteil nach einem der Ansprüche 5 bis 6, **dadurch gekennzeichnet, dass** der obere Randbereich (7) und der untere Randbereich (5) voneinander beabstandet sind.

8. Elektrodenbauteil nach einem der Ansprüche 1 bis 7, **dadurch gekennzeichnet, dass** an der zum Körper der Person weisenden Rückseite (8) des Elektrodenbauteils (1) mindestens eine Elektrode (9) mit Kontaktfläche (10) vorgesehen ist.

9. Elektrodenbauteil nach Anspruch 8, **dadurch gekennzeichnet, dass** an dem Elektrodenbauteil zwei Elektroden vorgesehen sind.

10. Elektrodenbauteil nach einem der Ansprüche 1 bis 9, **dadurch gekennzeichnet, dass** das Elektrodenbauteil (1), insbesondere gemeinsam mit einem weiteren Elektradenbauteil (11), in elektrischer Verbindung zu einer Senderelektronik (12) steht.

11. Elektrodenbauteil nach Anspruch 10, **dadurch gekennzeichnet, dass** das Elektrodenbauteil (1, 11) gemeinsam mit der in einem Gehäuse (13) angeordneten Senderelektronik (12) eine Mess- und Übertragungseinheit (14) ausbildet.

12. Elektrodenbautell nach Anspruch 10 oder 11, **dadurch gekennzeichnet, dass** sich das Elektrodenbauteil (1, 11) teilweise parallel zur Senderelektronik (12) bzw. zum Gehäuse (13) erstreckt.

13. Elektrodenbauteil nach Anspruch 12, **dadurch gekennzeichnet, dass** sich das Elektrodenbauteil (1, 11), insbesondere die Vorderseite (3), in radialer Richtung bezogen auf eine Längsachse (A) beabstandet zur Senderelektronik (12) bzw. zum Gehäuse (13) erstreckt.

14. Elektrodenbauteil nach Anspruch 13, **dadurch gekennzeichnet, dass** im Abstand zwischen Elektrodenbauteil (1, 11), insbesondere zwischen der Vorderseite (3), und der Senderelektronik (12) bzw. dem Gehäuse (13) das an der Person angeordnete Umfangsband (2) im Spannungszustand entlanggeführt ist und an der zum Körper der Person hinweisenden Rückseite (16) des Gehäuses (13) anliegt.

15. Elektrodenbauteil nach einem der Ansprüche 1 bis 14, **dadurch gekennzeichnet, dass** das Umfangsband (2) aus einem textilen, elastischen Material besteht.

16. Elektrodenbauteil nach einem der Ansprüche 1 bis 15, **dadurch gekennzeichnet, dass** das Elektrodenbauteil (1, 11) hinsichtlich seiner Trag-, Befestigungs-, Halte- und Führungsfunktion ausübenden Teile aus einem nichtleitenden Kunststoff besteht und dass die dem Elektrodenbauteil (1, 11) zugeordnete Elektrode (9) aus elektrisch leitendem Kunststoff, insbesondere aus ABS, oder aus Metall besteht.

17. Elektrodenbauteil nach einem der Ansprüche 12 bis 17, **dadurch gekennzeichnet, dass** das Gehäuse (13) aus festem Kunststoff besteht.

18. Verfahren zur Befestigung eines Elektrodenbauteils nach einem der Ansprüche 1 bis 17, am Körper einer Person mittels eines gespannten Umfangsbandes, wobei die mit mindestens einer Elektrode mit Kontaktfläche ausgestattete Rückseite des Elektrodenbauteils am Körper einer Person anliegt, umfassend die Schritte,
dass zunächst das Umfangsband um den Körper der Person gespannt wird und
dass danach das Elektrodenbauteil am Umfangsband eingehängt wird,
wobei das Umfangsband das Elektrodenbauteil festklemmt,

19. Verfahren nach Anspruch 18, **dadurch gekennzeichnet, dass** eine aus zwei Elektrodenbauteilen und einer Senderelektronik gebildete Mess- und Übertragungseinheit am Umfangsband eingehängt wird.

## Claims

1. An electrode component for measuring a person's heart rate, having fastening means (4, 5, 6, 7) for detachable attachment to a circumferential band (2), in particular in a chest belt, whereby the fastening means (4, 5, 6, 7) are an integral part of the electrode component (1), and whereby the circumferential band (2) arranged on the person is directly in contact with the front side of the electrode component (1) facing away from the person's body and securing it in the stressed state of the band,
**characterized in that**
the electrode component (1) is designed as an L-profile and/or as a C-profile in at least some areas.

2. The electrode component according to Claim 1,
**characterized in that**
the circumferential band (2) is guided in at least some areas by the electrode component (1).

3. The electrode component according to Claim 1 or 2,
**characterized in that**
the lower leg (4) of the L-profile or the C-profile has a lower edge area (5) that extends approximately parallel to the front side (3) of the electrode component (1).

4. The electrode component according to Claim 3,
**characterized in that**
the longitudinal dimension of the lower edge area (5) constitutes a portion of the total length (L) of the electrode component (1).

5. The electrode component according to any one of Claims 1 to 4,
**characterized in that**
the upper leg (6) of the C-profile has an upper edge area (7) extending approximately parallel to the front side (3) of the electrode component (1).

6. The electrode component according to Claim 5,
**characterized in that**
the length dimension of the upper area (7) constitutes a part of the total length (L) of the electrode component (1).

7. The electrode component according to any one of Claims 5 to 6,
**characterized in that**
the upper edge area (7) and the lower edge area (5) are spaced a distance apart from one another.

8. The electrode component according to any one of Claims 1 to 7,
**characterized in that**
at least one electrode (9) is provided with a contact surface (10) on the back side (8) of the electrode component (1) facing the person's body.

9. The electrode component according to Claim 8,
**characterized in that**
two electrodes are provided on the electrode component.

10. The electrode component according to any one of Claims 1 to 9,
**characterized in that**
the electrode component (1) is electrically connected to an electronic transmitter unit (12), in particular jointly with another electrode component (11).

11. The electrode component according to Claim 10,
**characterized in that**
the electrode component (1, 11) forms a measurement and transmission unit (14) together with the electronic transmitter (12) arranged in a housing (13).

12. The electrode component according to Claim 10 or 11,
**characterized in that**
the electrode component (1, 11) extends partially parallel to the electronic transmitter (12) and/or to the housing (13).

13. The electrode component according to Claim 12,
**characterized in that**
the electrode component (1, 11), in particular the front side (3), extends in the radial direction, based on a longitudinal axis (A), at a distance from the electronic transmitter (12) and/or from the housing (13).

14. The electrode component according to Claim 13,
**characterized in that**
the circumferential band (2) arranged on the person is guided in a stretched state at a distance between the electrode component (1, 11), in particular between the front side (3) and the electronic transmitter (12) and/or the housing (13) and is in contact with the back side (16) of the housing (13) facing the person's body.

15. The electrode component according to any one of Claims 1 to 14,
**characterized in that**
the circumferential band (2) is made of an elastic textile material.

16. The electrode component according to any one of Claims 1 to 15,
**characterized in that**
the electrode component (1, 11) consists of a nonconductive plastic with regard to its parts which have a carrying function, a fastening function, a holding function and a guidance function, and the electrode (9) assigned to the electrode component (1, 11) consists of an electrically conductive plastic, in particular ABS or metal.

17. The electrode component according to any one of the Claims 12 to 17,
**characterized in that**
the housing (13) is made of a solid plastic.

18. The method for fastening an electrode component according to any one of Claims 1 to 17 on a person's body by means of a stretched circumferential band, whereby the back side of the electrode component furnished with at least one electrode with a contact face is in contact with a person's body, comprising the steps whereby first the circumferential band is stretched around the person's body and then the electrode component is suspended from the circumferential band, whereby the circumferential band securely attaches the electrode component.

19. The method according to Claim 18,
**characterized in that**
a measurement and transmission unit formed by two electrode components and an electronic transmitter is suspended from the circumferential band.

## Revendications

1. Ensemble d'électrode pour la mesure de fréquence cardiaque d'une personne, comportant un moyen de fixation (4, 5, 6, 7) pour la fixation amovible sur une sangle (2) faisant le tour du corps, notamment une sangle de poitrine, dans lequel le moyen de fixation (4, 5, 6, 7) est un composant intégral de l'ensemble d'électrode (1) et dans lequel la sangle (2) faisant le tour du corps de la personne repose en l'état tendu sur le côté avant (3) de l'ensemble d'électrode (1) qui se détourne du corps de la personne et cale celui-ci,
**caractérisé en ce que**
la section transversale de l'ensemble d'électrode (1) est configurée au moins sur des portions comme un profilé en L et/ou un profilé en C.

2. Ensemble d'électrode selon la revendication 1, **caractérisé en ce que** la sangle faisant le tour du corps (2) est guidée au moins sur des portions par l'ensemble d'électrode (1).

3. Ensemble d'électrode selon la revendication 1 ou 2, **caractérisé en ce que** le pan inférieur (4) du profilé en L ou en C présente une zone de bord inférieure (5), qui s'étend approximativement parallèlement au côté avant (3) de l'ensemble d'électrode (1).

4. Ensemble d'électrode selon la revendication 3, **caractérisé en ce que** la dimension longitudinale de la zone de bord inférieure (5) constitue une partie de la longueur totale (L) de l'ensemble d'électrode (1).

5. Ensemble d'électrode selon une des revendications 1 à 4, **caractérisé en ce que** le pan supérieur (6) du profilé en C présente une zone de bord supérieure (7), qui s'étend approximativement parallèlement au côté avant (3) de l'ensemble d'électrode (1).

6. Ensemble d'électrode selon la revendication 5, **caractérisé en ce que** la dimension longitudinale de la zone de bord supérieure (7) constitue une partie de la longueur totale (L) de l'ensemble d'électrode (1).

7. Ensemble d'électrode selon une des revendications 5 à 6, **caractérisé en ce que** la zone de bord supérieure (7) et la zone de bord inférieure (5) sont espacées l'une de l'autre.

8. Ensemble d'électrode selon une des revendications 1 à 7, **caractérisé en ce que** sur le côté arrière (8) de l'ensemble d'électrode (1) tourné vers le corps de la personne, au moins une électrode (9) avec une surface de contact (10) est prévue.

9. Ensemble d'électrode selon la revendication 8, **caractérisé en ce que** deux électrodes sont prévues sur l'ensemble d'électrode.

10. Ensemble d'électrode selon une des revendications 1 à 9, **caractérisé en ce que** l'ensemble d'électrode (1), notamment conjointement à un autre ensemble d'électrode (11) est en liaison électrique avec une installation électronique d'émetteur (12).

11. Ensemble d'électrode selon la revendication 10, **caractérisé en ce que** l'ensemble d'électrode (1, 11) forme conjointement à l'installation électronique d'émetteur (12) disposée dans un boîtier (13) une unité de mesure et de transmission (14).

12. Ensemble d'électrode selon la revendication 10 ou 11, **caractérisé en ce que** l'ensemble d'électrode (1, 11) s'étend partiellement parallèlement à l'installation électronique d'émetteur (12), respectivement au boîtier (13).

13. Ensemble d'électrode selon la revendication 12, **caractérisé en ce que** l'ensemble d'électrode (1, 11), notamment le côté avant (3), s'étend dans la direction radiale par rapport à un axe longitudinal (A) en étant espacé de l'installation électronique d'émetteur (12), respectivement du boîtier (13).

14. Ensemble d'électrode selon la revendication 13, **caractérisé en ce que** la sangle (2) faisant le tour du corps de la personne est étirée en l'état tendu dans l'espacement entre l'ensemble d'électrode (1, 11), notamment entre le côté avant (3), et l'installation électronique d'émetteur (12), respectivement le boîtier (13) et repose sur le côté arrière (16) du boîtier (13) tourné vers le corps de la personne.

15. Ensemble d'électrode selon une des revendications 1 à 14, **caractérisé en ce que** la sangle faisant le tour du corps (2) est constituée d'un matériau textile élastique.

16. Composant d'électrode selon une des revendications 1 à 15, **caractérisé en ce que** l'ensemble électronique (1, 11) est constitué d'un plastique non conducteur en ce qui concerne ses pièces exerçant une fonction de portance, fixation, maintien et guidage et **en ce que** l'électrode (9) coordonnée à l'ensemble d'électrode (1, 11) est constituée d'un plastique électriquement conducteur, notamment de l'ABS, ou de métal.

17. Ensemble d'électrode selon une des revendications 12 à 17, **caractérisé en ce que** le boîtier (13) est constitué d'un plastique solide.

18. Procédé de fixation d'un ensemble d'électrode selon une des revendications 1 à 17, sur le corps d'une personne au moyen d'une sangle faisant le tour du corps tendue, moyennant quoi le côté arrière de l'ensemble d'électrode équipé d'au moins une électrode avec une surface de contact repose sur le corps d'une personne, comprenant les étapes consistant en ce que
d'abord, la sangle faisant le tour du corps est tendue autour du corps de la personne et ensuite l'ensemble d'électrode est suspendu sur la sangle, moyennant quoi la sangle faisant le tour du corps cale l'ensemble d'électrode.

19. Procédé selon la revendication 18, **caractérisé en ce que** une unité de mesure et de transmission formée par deux ensembles d'électrodes et une installation électronique d'émetteur est suspendue sur la sangle faisant le tour du corps.
